# EUROPEAN PATENT SPECIFICATION

(11) **EP 0 526 908 B1**
(45) Date of publication and mention of the grant of the patent: **04.09.1996**
(21) Application number: 92116814.2
(22) Date of filing: 11.05.1989
(51) Int. Cl.: C07C 19/12, C07C 17/10

(54) **Process for production of 1,1,1-trifluoro-2,2-dichloroethane**
Verfahren zur Herstellung von 1,1,1-Trifluoro-2,2-Dichlorethan
Procédé pour la production de 1,1,1,-trifluoro-2,2-dichloroéthane

(30) Priority: 17.05.1988 JP 120067/88
(43) Date of publication of application: 10.02.1993
(62) Divisional of application: 89108494.9
(73) Proprietor: DAIKIN INDUSTRIES, LIMITED, Osaka-shi, Osaka-fu (JP)
(72) Inventor: Furutaka, Yasuhisa, Takatsuki-shi, Osaka-fu (JP); Homoto, Yukio, Katano-shi, Osaka-fu (JP); Honda, Tsunetoshi, 203, Kobayashi Haitsu, Ageo-shi, Saitama-ken (JP)
(74) Representative: Hansen, Bernd, Dr. Dipl.-Chem.

(56) References cited:
- US-A- 4 060 469
- US-A- 4 145 368

## Description

The present invention relates to a process for production of 1,1,1-trifluoro-2,2-dichloroethane. Particularly, the present invention relates to a process for 1,1,1-trifluoro-2,2-dichloroethane comprising chlorination of 1,1,1-trifluoro-2-chloroethane with chlorine gas in the absence of a catalyst.

### Description of the Related Art

1,1,1-Trifluoro-2,2-dichloroethane is expected to replace trichlorofluoromethane, since it does not decompose ozone in the stratosphere. Then, it is desired to develop an economical process for the production of 1,1,1-trifluoro-2,2-dichloroethane.

Several processes for the production of 1,1,1-trifluoro-2,2-dichloroethane have been already proposed. For example, Czechoslovakian Patent No. 136,735 and Japanese Patent Kokai Publication No. 222038/1983 describe a process comprising reduction of 1,1,1-trifluoro-2,2,2-trichloroethane. Japanese Patent Publication No. 27375/1986 describes a process comprising isomerization of 1,1,2-trifluoro-1,2-dichloroethane. U.S. Patent No. 3,755,477 describes a process comprising fluorination of ethylene tetrachloride. Japanese Patent Kokai Publication No. 82711/1978 describes a process comprising photo chlorination of 1,1,1-trifluoro-2-chloroethane. McBee describes a process comprising chlorination of 1,1,1-trifluoroethane in J. Ind. Eng. Chem., 39, 409, (1947).

However, from the points of view of the economical production, the above known processes for the production of 1,1,1-trifluoro-2,2-dichloroethane are not necessarily suitable since yield and selectivity through such the process are not high enough.

McBee describes that 1,1,1-trifluoroethane, which is a similar compound to a starting material for the production of 1,1,1-trifluoro-2,2-dichloroethane, reacts at the reaction temperature of 485 °C with chlorine at the molar ratio of 1 : 1. Under these conditions, the conversion of 1,1,1-trifluoroethane is 50 %. The product through this process contains 22.5 % of 1,1,1-trifluoro-2,2-dichloroethane and 41.8 % of 1,1,1-trifluoro-2,2,2-trichloroethane. It is reported that judging from the fact that the amounts of the compounds of which all hydrogen atoms are replaced with chlorine atoms have been increased, the chlorination rate increases as the chlorination proceeds, that is, as the number of hydrogen atoms replaced with chlorine atoms increases.

Accordingly, the above report indicates that the molar ratio of 1,1,1-trifluoro-2,2,2-trichloroethane to 1,1,1-trifluoro-2,2-dichloroethane is more than 1, that is, 1,1,1-trifluoro-2,2,2-trichloroethane is always produced more than 1,1,1-trifluoro-2,2-dichloroethane.

US-A-4 145 368 teaches that the vapour phase thermal chlorination of 1,1,1-trifluoro-2-chloroethane (133A) to obtain the product 1,1,1-trifluoro-2,2-dichloroethane (123) is possible without a catalyst.

US-A-4 060 469 discloses the preparation of 123 by contacting 133A with less than a molar equivalent of chlorine and carrying out photochemical chlorination at a temperature of 5-175°C.

### SUMMARY OF THE INVENTION

It is, therefore, an object of the present invention to provide a commercially advantageous process for the production of 1,1,1-trifluoro-2,2-dichloroethane which overcomes the problems described above.

According to the present invention, there is provided a process for the production of 1,1,1-trifluoro-2,2-dichloroethane comprising the step of chlorinating 1,1,1-trifluoro-2-chloroethane with chlorine gas in the absence of a catalyst characterised in that the molar ratio of chlorine gas to 1,1,1-trifluoro-2-chloroethane is in the range 0.05-0.5, and in that the chlorination is carried out at a temperature of 350-450° C.

With the process of the present invention, the production ratio of 1,1,1-trifluoro-2,2-dichloroethane to 1,1,1-trifluoro-2,2,2-trichloroethane is about 1 : 0.3 at about 50 % conversion of 1,1,1-trifluoro-2-chloroethane and such a high production ratio cannot be expected from the report of McBee. Then, it becomes possible to produce 1,1,1-trifluoro-2,2-dichloroethane economically.

### DETAILED DESCRIPTION OF THE INVENTION

1,1,1-Trifluoro-2-chloroethane, as a starting material in the present process, can be easily produced by fluorination of trichloroethylene with anhydrous hydrogen fluoride in a liquid or gas phase.

The chlorination of the present invention can proceed with high selectivity even though it is carried out in the absence of a catalyst.

The reaction temperature of the chlorination of the present process is in the range of from 350 to 450 °C, for example 400 °C. The reaction is usually performed under the atmospheric pressure, although the present chlorination can be performed under pressure.

The molar ratio of chlorine gas to 1,1,1-trifluoro-2-chloroethane is controlled in the range of from 0.05 to 0.5, particularly from 0.1 to 0.4 by taking account of the selectivity to 1,1,1-trifluoro-2,2-dichloroethane.

In the present process, any type of reactor, for example a tube reactor, can be used.

In the process of the present invention, the reactor such as a tube reactor is heated to a preselected temperature dependent on the reaction temperature, for example, in an electrical furnace. Then, 1,1,1-trifluoro-2-chloroethane and chlorine gas are supplied to the reactor to start the chlorination. The exit gas from the reactor is generally collected after water washing and drying steps.

In order to improve the conversion of 1,1,1-trifluoro-2-chloroethane, it is advantageous to recycle the unreacted 1,1,1-trifluoro-2-chloroethane to the reactor which is recovered from the top of a purification apparatus for purifying the produced gas.

It is advantageous to use an inert packing such as Raschig ring in the reactor from the view of better mixing of the gas and better heat transfer.

Present invention will be hereinafter explained further in detail by following examples.

### Reference Example 1

A tube reactor made of Hastelloy C (20 mm in inner diameter, 400 mm in length) was filled with 50 ml of spherical support particles (from 2 to 4 mm in diameter) made of AlF₃ carrying CuCl₂ in the molar ratio of 0.03 to AlF₃, and then heated to 370 °C in a nitrogen stream. After stopping the supply of nitrogen, 1,1,1-trifluoro-2-chloroethane and chlorine gas were supplied to the reactor at the flow rate of 100 ml/min. and 50 ml/min. (based on the standard conditions), respectively. The contact time based on the average residence time was about 8.5 seconds.

The exit gas from the tube reactor was collected after the water washing and the drying steps, and then analyzed with a gas chromatography. The conversion of 1,1,1-trifluoro-2-chloroethane was 45 % and the selectivity to 1,1,1-trifluoro-2,2-dichloroethane was 75 %.

### Examples 1-3

Using the same apparatus as Reference Example 1, the process of the present invention was carried out without a catalyst. In these examples, 1,1,1-trifluoro-2-chloroethane was reacted with chlorine gas at the temperature of 400 °C with the ratio of the flow rate thereof as shown in Table 1. The exit gas from the reactor was analyzed as in Reference Example 1.

The results are also shown in Table 1.

**Table 1**

| | Example 1 | Example 2 | Example 3 |
|---|---|---|---|
| Flow rate of 1,1,1-trifluoro-2-chloroethane | 90 ml/min. | 80 ml/min. | 70 ml/min. |
| Flow rate of chlorine gas | 10 ml/min. | 20 ml/min. | 30 ml/min. |
| Conversion of 1,1,1-trifluoro-2-chloroethane | 10 % | 22 % | 40 % |
| Selectivity to 1,1,1-trifluoro-2,2-dichloroethane | 91 % | 86 % | 77 % |

### Comparative Example

1,1,1-Trifluoro-2-chloroethane and chlorine gas were supplied to a glass tube reactor (30 mm in inner diameter, 200 mm in length) made of Pyrex Glass® (commercially available from Corning Glass Works, U.S.) under the illumination of a high pressure mercury lamp of 100 wattages. Their flow rates were 100 ml/min. and 50 ml/min., respectively. The residence time in the reactor was about 60 seconds. The temperature in the reactor was 30 °C.

The produced gas discharged from the reactor was analyzed with the gas chromatography after water washing. The conversion of 1,1,1-trifluoro-2-chloroethane was 5 % and the selectivity to 1,1,1-trifluoro-2,2-dichloroethane was 10 %. The rest of the product was 1,1,1-trifluoro-2,2,2-trichloroethane.

## Claims

1. A process for the production of 1,1,1-trifluoro-2,2-dichloroethane comprising the step of chlorinating 1,1,1-trifluoro-2-chloroethane with chlorine gas in the absence of a catalyst characterised in that the molar ratio of chlorine gas to 1,1,1-trifluoro-2-chloroethane is in the range 0.05-0.5, and in that the chlorination is carried out at a temperature of 350-450° C.

## Patentansprüche

1. Verfahren zur Herstellung von 1,1,1-Trifluor-2,2-dichlorethan, umfassend den Schritt der Chlorierung von 1,1,1-Trifluor-2-chlorethan mit Chlorgas in Abwesenheit eines Katalysators,
dadurch **gekennzeichnet,** daß
das Molverhältnis von Chlorgas zu 1,1,1-Trifluor-2-chlorethan im Bereich von 0,05 bis 0,5 liegt und daß die Chlorierung bei einer Temperatur von 350 bis 450°C ausgeführt wird.

## Revendications

1. Procédé pour la production de 1,1,1-trifluoro-2,2-dichloroéthane comprenant l'étape de chloration de 1,1,1-trifluoro-2-chloroéthane avec du gaz de chlore en l'absence d'un catalyseur, caractérisé en ce que le rapport molaire du gaz de chlore au 1,1,1-trifluoro-2-chloroéthane se trouve dans l'intervalle de 0,05-0,5 et en ce qu'on réalise la chloration à une température de 350-450°C.
